# EUROPEAN PATENT SPECIFICATION

(11) **EP 1 346 227 B1**
(45) Date of publication and mention of the grant of the patent: **30.06.2004**
(21) Application number: 01985898.4
(22) Date of filing: 18.12.2001
(51) Int. Cl.: G01N 33/68, C12Q 1/37, G01N 33/53

(54) **DETECTION OF ADVANCED GLYCATION ENDPRODUCTS IN A CEREBROSPINAL FLUID SAMPLE**
VERFAHREN ZUM NACHWEIS VON FORTGESCHRITTENEN GLYZIERUNGSENDPRODUKTE IN ZEREBROSPINALFLÜSSIGKEIT
DETECTION DE PRODUITS TERMINAUX DE GLYCOSYLATION APPROFONDIE DANS UN ECHANTILLON DE LIQUIDE CEPHALORACHIDIEN

(30) Priority: 21.12.2000 EP 00127948; 13.02.2001 EP 01102826
(43) Date of publication of application: 24.09.2003
(73) Proprietor: Roche Diagnostics GmbH, 68305 Mannheim (DE); F. HOFFMANN-LA ROCHE AG, 4002 Basel (CH)
(72) Inventor: KIENTSCH-ENGEL, Rosemarie, 82340 Feldafing (DE); STAHL, Peter, 82347 Bernried (DE); VON DER ELTZ, Herbert, 82362 Weilheim (DE)
(86) International application number: PCT/EP2001/014963
(87) International publication number: WO 2002/050548

(56) References cited:
- WO-A-95/20979
- US-A- 4 658 022
- US-A- 5 610 076
- SHUVAEV, V.V., ET AL: "Increased protein glycation in crebrospinal fluid of Alzheimer's disease" NEUROBIOLOGY OF AGING, vol. 22, 1 May 2001 (2001-05-01), pages 397-402, XP001014392

## Description

The present invention relates to the measurement of advanced glycation endproducts in cerebrospinal fluid, and especially to the assessment and monitoring of neurodegenerative disorders based on such measurement.

Alzheimer's Disease (AD) is the most common neurodegenerative disorder characterized by progressive dementia that ultimately leads to death. It is also one of the most devastating brain diseases and a severe medical, psychological and economic problem in modern societies. For example, there are about 600.000 patients suffering from Alzheimer's Disease in Japan.

Despite the huge number of cases and the tremendous costs to healthcare systems the appropriate diagnosis of Alzheimer's Disease is a major challenge to the clinician in the field.

At least three different approaches all of some, despite limited utility are available.

First, there are so-called mental or cognitive examinations. Such examinations are very difficult to standardize and to transfer from one institution to the other. One of the most widely used examination follows the instructions given by the National Institute of Neurological and Communicative Disorders and Strokes-Alzheimer's Disease and Related Disorders Association, the so-called NINCDS-ADRDA-criteria (McKhann, G., et al., Neurology 34 (1984) 939-944).

Second, there are physical approaches to diagnose Alzheimer's Disease which are largely based on metabolic activities of the brain investigated. Such physical methods include for example near-infra-red-spectroscopy (NIRS) and PET. These methods may for example be used to investigate the level of oxygenation of hemoglobin in the brain as for example described by C. Hock, et al., Brain Research 755 (1997) 293-303.

Third, to be mentioned is the approach to diagnose Alzheimer's Disease by biological markers. The major problem and obstacle to the use of biological markers is the fact that Alzheimer's Disease is rather slowly progressing. It is assumed that there is quite a long preclinical phase, i.e., a phase in which the disease starts and progresses without leading to the manifestation of clearly visible or diagnostically accessible symptoms or markers. Even after clinical onset the progress in disease is slow and it takes five to ten years until a patient dies due to the disease (C. Hock, Neurobiology of Aging 19 (1998) 149-155). Biological markers, nonetheless, represent extremely attractive tools to better detect, monitor and diagnose AD. They can be collected repetitively, their measurement is comparatively cheap and easy to perform and the result of such measurement is not as difficult to standardize as for example mental examinations.

Several protein markers have attracted major attention during the past ten years, for example, the protein known as amyloid precursor protein (APP) and especially the breakdown product of this APP that is known as amyloid β-peptide (Aβ) (Weidemann A., et al., Cell 57 (1989) 115-126), the tau-protein and the apolipoprotein E4.

Only apolipoprotein E4 is diagnosed from blood. The tau-protein or its variants containing different degrees of phosphorylation, as well as the various forms of Aβ usually are tested in cerebrospinal fluid (CSF).

Advanced glycation endproducts (AGEs) in contrast are a hall-mark of diabetic disease and have been studied there extensively.

In the formation of AGEs in a first step glucose and other reducing sugars attach non-enzymatically to the amino groups of proteins in a concentration-dependent manner.

The non-enzymatic reaction between glucose and the free amino groups on proteins to form a stable amino, 1-deoxy ketosyl adduct, known as the Amadori product, has been shown to occur with hemoglobin, wherein a rearrangement of the amino terminus of the β-chain of hemoglobin by reaction with glucose forms an adduct and gives rise to a product known as hemoglobin A_{1c.} Similar reactions have also been found to occur with a variety of other body proteins, such as lens crystallin, collagen and nerve proteins (see Bunn et al., Biochem. Biophys. Res. Commun. 67 (1975)103-109; Koenig et al., J. Biol. Chem. 252 (1975) 2992-2997; Monnier and Cerami, Maillard Reaction in Food and Nutrition, ed. Waller, G. A., American Chemical Society (1983) 431-448; and Monnier and Cerami, Clinics in Endocrinology and Metabolism 11 (1982) 431-452).

Over time, these initial Amadori adducts can undergo secondary reactions like further oxidation, rearrangements, dehydrations and cross-linking with other protein groups, and finally accumulate as a family of complex structures referred to as AGEs. Substantial progress has been made towards the elucidation of the biological roles and clinical significance of advanced glycation endproducts, so that it is now acknowledged that many of the conditions heretofore attributed to the aging process or to the pathological effects of diseases such as diabetes, are attributable at least in part to the formation, accumulation and/or activity of AGEs *in vivo.*

Because the above mentioned secondary reactions occur slowly, proteins may accumulate significant amounts of Amadori products before accumulating a measurable amount of AGEs *in vivo.* AGEs may modify important biological molecules / structures, like receptors, membranes or enzymes. They can cause protein cross-linking, which in turn may reduce the structural and/or functional integrity of organs and organ parts, thus ultimately reducing or impairing organ function.

The advanced glycation process is particularly noteworthy in that it predominately effects proteins with long half-lives, e.g., such as collagen under conditions of relatively high sugar concentration, such as in diabetes mellitus. Numerous studies have suggested that AGEs play an important role in the structural and functional alteration which occurs in proteins during aging and in chronic disease.

A second physiological condition giving rise to AGE-modified biomolecules is summarized as oxidative stress. It is known that oxidative stress leads, amongst other events, to the formation of advanced glycation end-products and may be one of the patho-mechanism in neurodegenerative disorders, e.g. in AD (Behl, Chr., Progress in Neurobiology 57 (1999) 301-323). It is also known that striking similarities are found when e.g. comparing in *vitro* properties of Aβ and the so-called prion proteins known as hallmarks or pathogenic agents from diseases summarized as transmissible spongiform encephalopathies (TSE). Oxidative stress is of major importance in the pathogenesis of TSE, e.g. of bovine spongiform encephalopathie (BSE) in cattle and Creutzfeldt-Jacob-Disease (CJD) in humans (Kretzschmar, et al., Nature (1996) 380: 345-347). Oxidative stress phenomena thus trigger the formation of AGE in such neurodegenerative diseases like AD, BSE, or CJD.

Recently, antibody reagents have become available and methods have been described, which may be used in the reproducible detection of well-defined AGE-structures.

US 5,610,076 discloses the specific detection of hemoglobin carrying AGE-structures (Hb-AGE). The improvement described in this patent resides in the pre-treatment of samples with detergents and/or chaotropic reagents. The positive effects of such pre-treatment are explained by exposure of Hb-AGE epitopes that otherwise would not be accessible to anti-AGE antibodies.

US 5,698,197 describes a monoclonal antibody (4G9) reactive with in-vivo formed AGEs. In one embodiment this antibody is used in a sandwich type ELISA to detect Apo-B-AGE, IgG-AGE, collagen-AGE, serum AGE-peptides and proteins as well as urinary AGE-peptides and proteins. In order to perform these AGE-sandwich assays monoclonal antibody 4G9 or an immunoreactive fragment thereof is directly coated to the solid phase. Competitive immunoassay settings are also described using 4G9. BSA-AGE is used as antigen and coated to the solid phase of such competition type assays.

WO95/20979 discloses a method for estimating the extent of amyloidosis in a mammal comprising measuring the presence and amount of AGE-amyloid polypeptide in cerebrospinal fluid.

Whereas all the approaches in the field of Alzheimer's disease as discussed further above, like PET-scans, mental examinations, or certain biochemical markers may be used to some extend in the assessment of Alzheimer's Disease, there still is a tremendous need especially for improved and easy to use biochemical or biological markers to further improve the tools available in order to detect, diagnose or monitor neurodegerative diseases, like Alzheimer's Disease, BSE or CJD.

It has now surprisingly been found that the level of advanced glycation endproducts (AGE) in CSF-samples can be used with great advantage in the assessment and diagnosis of neurodegenerative diseases, like AD, BSE or CJD, especially when measures are taken to expose AGE-epitopes.

### Summary of the invention

The present invention discloses a method for assessment of neurodegenerative diseases, especially of Alzheimer's Disease (AD) and Creutzfeldt-Jacob-Disease (CJD) in humans and of bovine spongiform encephalopathy (BSE) in cattle, based on measurement of at least one advanced glycation endproduct (AGE) in a cerebrospinal fluid (CSF) sample, characterized in that, said CSF sample is pre-treated to expose AGE-epitopes, said pre-treatment resulting in protein denaturation.

It has been found that pre-treatment of cerebrospinal fluid that results in protein denaturation is an effective means of exposing AGE-epitopes. It has also been found that the level of AGE-epitopes, which can be exposed, is much higher in CSF obtained from patients diagnosed with Alzheimer's Disease, as compared to CSF-samples collected from controls.

In one embodiment a method according to the present invention is used to assess Alzheimer's Disease by comparing AGE-CSF-levels measured in a sample collected from a patient known to or suspected of having Alzheimer's Disease and to compare such levels to levels measured in parallel or known from control samples.

The method according to the present invention is used as well to monitor Alzheimer's Disease.

A further embodiment of the present invention is a method to expose AGE-epitopes as present in a CSF sample, said method comprising use of proteolytic enzymes, like esperase or proteinase K.

The use of methods according to the present invention in screening efforts for drugs affecting Alzheimer's Disease which influence the level of AGE in CSF is of course a major area of potential application representing another preferred embodiment of the present invention which is of major relevance to the field of AD.

### Detailed description of the invention

In a preferred embodiment the present invention relates to a method of measurement of at least one advanced glycation endproduct (AGE) in a cerebrospinal fluid (CSF) sample, characterized in that, said CSF sample is pre-treated resulting in protein denaturation. Such pre-treatment leads to the exposure of AGE-epitopes which are not recognized by a specific binding partner in another aliquot of the same sample without such pre-treatment.

Advanced glycation endproducts (AGEs) are a hall-mark of diabetic disease and have been studied there extensively.

As mentioned further above, AGEs are known and used as markers of diabetes.

It is therefore surprising, that AGE-structures contained in cerebrospinal fluid have now been found and demonstrated to be of major importance in the assessment of neurodegenerative diseases, such as AD, CJD or BSE. Even more surprising is the fact, that a pre-treatment resulting in protein denaturation of CSF fluid is required to expose additional AGE-epitopes and especially, that such exposure has different effects in CSF-samples collected from patients suspected or diagnosed with Alzheimer's Disease as compared to samples taken from healthy controls. Obviously, the CSF of patients with Alzheimer's Disease must contain a significant proportion of AGE-epitopes which can be exposed by appropriate treatments, whereas comparatively low levels of such exposable AGE-epitopes exist in CSF from healthy people.

As mentioned above, several quite different disease entities in the field of neurodegenerative diseases are characterized by loss of neurons, congnitive impairments and clinical manifestations of different type and nature. These neurodegenerative diseases comprise amongst others the Alzheimer's Disease, Parkinson's Disease and diseases grouped under TSEs. These TSEs include such different diseases of animals and humans like Scrapie, BSE, Kuru-kuru, Gerstmann-Sträussler-Scheinker-Syndrome and CJD. Oxidative stress is known as one of the factors also contributing to diseases like CJD or BSE (Kretzschmar, supra). These diseases now also have been found to exhibit changes in AGE-CSF levels.

A preferred embodiment according to the present invention is, therefore, a method for assessment of a neurodegenerative disease based on measurement of at least one advanced glycation endproduct in cerebrospinal fluid characterized in that said CSF-sample is pre-treated to expose AGE-epitope, said pre-treatement resulting in protein denaturation.

The term "AGE-epitope" is used to describe a structure which is the result of a non-enzymatic glycation plus oxidation process or as a result of reactions accompanied with oxidative stress which is bound or recognized by a specific binding partner especially by an antibody specifically reacting with this structure. One well-known example of such an AGE-epitope-specific binding partner is monoclonal antibody 4G9, as described in US 5,698,197.

There are many ways of "pre-treatment resulting in protein denaturation", some of which will be discussed in more detail below. Such pre-treatment may be performed completely separate from the test mixture, which is finally used to assess AGE-levels. It is, however, also possible to use rather short pre-incubations with a denaturing reagent. At its extremes, pre-treatment may be performed simultaneously to the analysis. It is, however, preferred that the CSF-sample is first pre-treated with an appropriate agent and/or method and thereafter, the level of AGE is measured using an appropriate assay, e.g. an immunoassay.

The phrase "exposed" is used to indicate that epitopes which are not accessible to a specific binding partner without pre-treatment are accessible (exposed) after appropriate treatment.

Out of the many methods possible to be used in the pre-treatment it has been found that such methods must result in protein denaturation. Such denaturation results for example in unfolding of globular protein structures and in dissociation of protein and/or peptide complexes. A method according to the present invention is performed by including a pre-treatment step which results in protein denaturation.

Out of the various methods and agents known in the art to perform such pre-treatment, it is preferred to use reagents selected from the group of detergents, chaotropic reagents and/or proteolytic enzymes. It is also possible to apply a method of heat treatment or a method of acid treatment to expose additional AGE-epitopes in a CSF-sample. All these reagents or methods are known to result in protein-unfolding and dissociation of protein and/or peptide aggregates.

AGE-epitopes usually represent rather small structures. Such epitopes may be masked or hidden within a protein or peptide molecule. One of the preferred ways to expose these epitopes is the use of proteolytic enzymes. A preferred embodiment according to the present invention is a method to expose AGE-epitopes as present in a CSF sample, said method comprising use of a proteolytic enzyme.

The use of the proteolytic enzyme may preferably be combined with the use of other pre-treatment methods. E.g., it is possible to first use a chaotropic denaturing agent and thereafter to apply a proteolytic enzyme. It is also a preferred embodiment of the present invention to use several proteolytic enzymes in combination.

It is possible to measure the level of various AGE-structures or AGE-epitopes which are present in CSF by different methods like HPLC (high performance liquid chromatography), MS (mass spectroscopy) or specific binding assays.

Immunological detection by specific antibodies is preferred. By standard immunoassay procedures, e.g. as described in relevant textbooks, like "Practice and theory of enzyme immunoassays" by Tijssen, Elsevier, Amsterdam, 1990 and many others, or as illustrated by the example given in the methods section, the level of the AGE structure under investigation is measured.

In a preferred method according to the present invention at least one proteolytic enzyme is used in the pre-treatment of a CSF-sample.

Theoretically, many different proteolytic enzymes may be used. However, it is preferred to use highly active and cheap enzymes, that are easy to handle. Examples for such enzymes are esperase and proteinase K. Use of proteinase K is most preferred.

Enzymatic pre-treatment with proteolytic enzymes may be performed under various conditions with respect to e.g. enzyme concentration, time or temperature used. It is preferred to standardize this step in order to not introduce additional variation into the overall measurement. Preferably conditions are chosen, which lead to maximum exposure of AGE-epitopes. E.g., in the detection of AGE-CML (an AGE-epitope comprising a structure derived from carboxy-methyl-lysine=CML), proteinase K is preferably used in a concentration from 0,3 to 5 mg/ml or also preferred from 0,5 to 2 mg/ml for preferably 0,5 to 24 hours, more preferred from 1 to 6 hours. It is also preferred to control for the temperature during enzymatic digestion. Temperature may e.g. be set to 25°C or 37°C. An elevated temperature like 37°C is preferred.

In order to better and more reliably assess the clinical meaning of an AGE-value, measured as described above, it is advantageous to compare the levels measured to levels known or obtained in parallel from one or more CSF-control samples. It is therefore preferred to use the above measurements in a method to assess a neurodegenerative disease, comprising comparing at least one AGE-CSF-level in a sample to be assessed to an AGE-CSF-level known or obtained from one or more CSF-control samples. The term "AGE-CSF" is used to indicate that an AGE from a CSF sample is investigated. Of course, it is also possible to compare the AGE-level measured in a clinical CSF-sample to the average value or cut-off value as obtained for said AGE in a large group of CSF-samples obtained from control individuals.

It is especially preferred to use a method to the present invention to assess a neurodegenerative disease selected from the group consisting of AD, CJD, and BSE. Most preferred the inventive methods are used to assess AD in humans or CJD in cattle.

It is further preferred to use a method according to the present invention for the purpose of diagnosing AD. For a diagnostic application usually the level of a marker of interest is carefully investigated in healthy individuals or for differential diagnosis in clinical samples, which are obtained from clinically related patients cohorts. From the values measured in these control samples the mean value as well as the standard deviations from this mean value are calculated. Based on these values a cut-off value which usually represents the mean plus or minus 2, or in some cases plus or minus 3 standard deviations is defined. Values measured in clinical samples which are above or below this cut-off value may be indicative or diagnostic of a certain disease. With such cut-off values established, the methods according to the present invention is used to diagnose Alzheimer's Disease. The use of the present invention to diagnose Alzheimer's Disease or to differentiate AD from other related neurological disorders therefore represents a further preferred embodiment of the present invention.

As mentioned above, Alzheimer's Disease is a rather slow progressing disorder. There are few, if any, means, to assess progressing or amelioration of Alzheimer's Disease and biochemical markers so far are of limited use in that respect.

AGE levels in CSF may change during the course of the disease as well as dependent on usage of drugs. Such changes now can be followed and measured over time, i.e. monitored. It represents a further preferred embodiment to use a method according to the present invention to monitor AD.

Treatment measures for Alzheimer's Disease are scarce and, only very few drugs are available to treat this important disease. Screening for new drugs is hampered by the fact that not many reliable biochemical markers are available. It is known that many AGE-structures are similar if not identical in animal species as well as in humans.

Based on the improved methods of measurement of CSF-AGE-levels according to this invention, it is now possible to analyze and investigate CSF-samples of laboratory animals or of patients in order to assess drug efficacy via changes in AGE-CSF-levels. It is now feasible to screen for drugs which are effecting AGE-CSF-levels and it is further preferred to use a method according to the present invention to screen for drugs in the field of mental disorders, especially AD which influence the level or affect the level of an AGE in CSF.

The following examples, references, and the figure are provided to aid the understanding of the present invention, the true scope of which is set forth in the appended claims. It is understood that modifications can be made in the procedures set forth without departing from the spirit of the invention.

### Description of the Figure

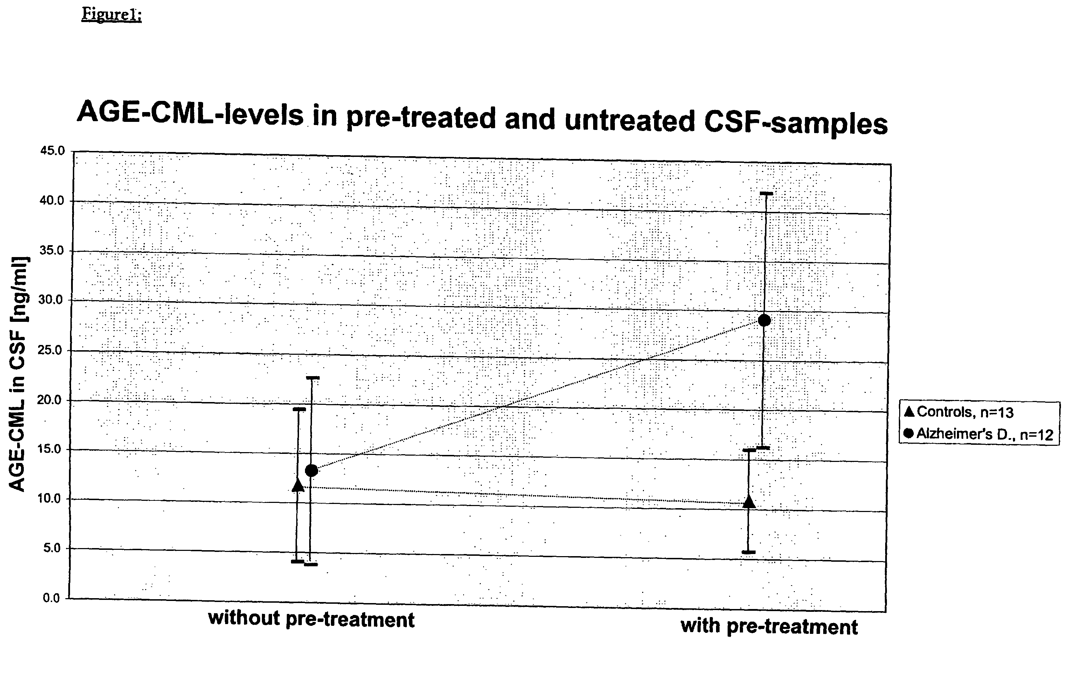

### Figure 1: AGE-CML-levels in pre-treated and untreated CSF samples

Cerebrospinal fluid samples from healthy controls and from patients with Alzheimer's Disease have been investigated with and with-out sample pre-treatment. As can be seen the levels of the AGE-structure carboxy-methyl-lysine (CML) as detected by monoclonal antibody 4G9 are approximately the same for both groups before treatment. However, after exposure of CML-epitopes by pre-treatment an increased AGE-CML-level is found with CSF samples from AD patients as compared to pre-treated CSF-samples from control individuals. The vertical bars indicate the standard deviation as calculated.

### Example 1

### Pre-treatment of cerebrospinal fluid

### 1.1 Buffer and reagents

### a) Buffer

10 mM TRIS
150 mM NaCl
0,001 % (w/v) N-methyl-iso-thiazolone
0,01 % (w/v) 2-chloro-acetamide
0,05 % (v/v) Tween® 20
pH 7,4

### b) Proteinase K

Roche Molecular Biochemicals, Cat. No. 236 608
Solution of 13 mg/ml in the above buffer solution is used.

### c) PMSF (= Phenylmethylsulfonyl Fluoride)

Roche Molecular Biochemicals, Cat. No. 236 608
PMSF, which is dissolved in Ethanol to yield 100 mMol/l is diluted shortly before use 1+50 into the above buffer.

### 1.2 Digestion of CSF

To 60 µl of CSF 5 µl of proteinase K-solution (final concentration 1 mg/ml) is added. Reagents are mixed and incubated for 3 hours at 37 °C. To inhibit the proteinase K activity 65 µl of PMSF are added (final concentration 1 mM) and the reagent mixture is further incubated for 30 to 60 minutes.

Thereafter, the pre-treated sample is ready for AGE-CML-determination.

### Example 2

### Measurement of AGE-CML

### 2.1 Reagents and solutions

### a) Incubation buffer

A commercially available incubation buffer (Fibrin-monomer-assay from Roche Diagnostics GmbH, Product No. 565 440) has been used.

### b) Washing solution

10 mM TRIS
150 mM NaCl
0,001 % (w/v) N-methyl-iso-thiazolone
0,01 % (w/v) 2-chloro-acetamide
0,05 % (v/v) Tween® 20
pH 7,4

### c) Substrate:

The commercially available Enzymun® -Assay substrate (Roche Diagnostics GmbH, Product No. 1 295 250) has been used.

### 2.2. Reagents used to perform the assay

### a) Bi-BSA-AGE

Bovine-Serum-Albumin (BSA, Calbiochem, Order no. 12657) has been subjected to advanced glycation *in vitro.* For this purpose, BSA-AGE was obtained after incubation of BSA (50 mg/ml in 50 mM potassium phosphate, 150 mM sodium chloride, 20 µM copper sulfate, pH 7,4) with D-glucose (0,5 M) for 3 weeks at 35 °C and then dialysed against 100 mM potassium phosphate buffer, 100 mM sodium chloride, pH 8,0.

For biotinylation 50 mg BSA-AGE was incubated with 3,4 mg D-Biotinoyl-[epsilon]-aminocaproic acid-N-hydroxysuccinimide ester for 90 min and the reaction was stopped by addition of lysine-monochloride to yield a final concentration of 10 mM. Finally the solution was dialysed over night against phosphate buffered saline, 50 mM potassium phophate, 150 mM sodium chloride, pH 7,5. The biotinylated product is called "Bi-BSA-AGE".

Bi-BSA-AGE is used as a 1 µg/ml solution in the above incubation buffer.

### b) Standards

6-(N-carboxymethylamino)caproic acid, di-sodium salt (a CML-type synthetic molecule with MW 233) is used as standard material. Solutions containing 0/6,25/12,5/25/50/100 ng/ml of standard are prepared by appropriate dilution of this material in incubation buffer.

### c) Detection reagent

Monoclonal antibody 4G9, which is known to react with Carboxymethyl lysine (CML) structures, e.g, like the above mentioned 6-(N- carboxymethylamino) caproic acid, has been purified and conjugated with horseradish peroxidase (POD) according to standard procedures. The activity of such conjugate can be expressed in terms of peroxidase units. In the assay as described below, the monoclonal antibody POD-conjugate is diluted in incubation buffer to obtain roughly 90 mU/ml of POD-activity.

### 2.3 Streptavidin-coated solid phase

BSA-Biotin was prepared according to EP 0 331 127 (example 1), dissolved and diluted to 10 µg/ml in 50 mM potassium phosphate, pH 7,4 and 300 µl, each, incubated in the wells of a microtiter plate (NUNC Maxisorp® ) for 5 h at room temperature. The plate was emptied and refilled with 300 µl/well homogeneously crosslinked streptavidine (according to example 2 of EP 0 331 127) 10 µg/ml in 50 mM potassium phosphate, pH 7,4. After 18 h incubation at room temperature the wells were emptied and refilled with a solution of 9 g/l sodium chloride, 10 g/l Dextran T40 and 3 g/l BSA (300 µl/well). After 30 min the plates were completely emptied, dried during 3 h at 25 °C, 2% relative humidity, and sealed in an airtight bag.

### 2.4 Immunoassay for AGE-CML

The wells of a streptavidin-coated micro titer plate are incubated with Bi-BSA-AGE (1 µg/ml). 100 µl/well are incubated at room temperature for 1 hour. Any unbound Bi-BSA-AGE is removed by washing all wells with 3 x 300 µl washing solution.

Sample and peroxidase-conjugated monoclonal antibody are co-incubated. 50 µl of sample (either proteinase K pre-treated CSF, untreated CSF, or standard material) is added to the wells immediately followed by 50 µl of conjugate solution as prepared above. This mixture is incubated for 1 hour at room temperature.

Non-bound reagents are removed by washing, as described above.

Bound peroxidase is detected by standard substrate reaction. For this purpose 100 µl of substrate solution are added per well and incubated for roughly 30 minutes at room temperature. Peroxidase activity is measured via the change in substrate at a wavelength of 405 nm.

During all incubation steps the reaction mixture in the wells of the micro titer plate is gently moved using a plate shaker device.

Concentration of AGE-CML in CSF is extrapolated from the standard curve according to standard procedures.

Representative results, as obtained with untreated CSF-samples or samples pre-treated as described above are summarized in tables 1/1 and 1/2. Table 1/1 gives the individual data measured and table 2/2 is a statistical evaluation thereof.

**Table 1/2:**

| AGE-CML in CSF-samples | | |
|---|---|---|
| Diagnosis | CML without pre-treatment [ng/ml] | CML with pre-treatment [ng/ml] |
| Control | 16.6 | 12.0 |
| Control | 7.2 | 11.9 |
| Control | 10.0 | 8.5 |
| Control | 33.8 | 19.1 |
| Control | 14.1 | 20.5 |
| Control | 8.8 | 6.0 |
| Control | 11.4 | 9.8 |
| Control | 11.0 | 3.7 |
| Control | 0.0 | 3.1 |
| Control | 12.0 | 9.6 |
| Control | 9.5 | 11.0 |
| Control | 11.1 | 13.5 |
| Control | 7.4 | 12.3 |
| AD | 3.6 | 22.9 |
| AD | 4.8 | 16.4 |
| AD | 10.3 | 19.1 |
| AD | 25.6 | 29.5 |
| AD | 30.4 | 54.5 |
| AD | 26.1 | 47.8 |
| AD | 13.8 | 14.2 |
| AD | 0.3 | 17.4 |
| AD | 10.2 | 38.1 |
| AD | 13.6 | 34.2 |
| AD | 9.2 | 22.6 |
| AD | 10.9 | 32.0 |

**Table 2/2:**

| Mean values (MW) and standard deviation (SD) | | |
|---|---|---|
| controls, n=13 | w/o PT | w PT |
| MW | 11.8 | 10.8 |
| SD | 7.7 | 5.1 |
| AD, n=12 | w/o PT | w PT |
| MW | 13.2 | 29.1 |
| SD | 9.5 | 12.8 |

| | | |
|---|---|---|
| (w/o PT) = without pre-treatment | | |
| (w PT) = with pre-treatment | | |

### List of References

Behl, Chr., Progress in Neurobiology 57 (1999) 301-323
Bunn et al., Biochem. Biophys. Res. Commun. 67 (1975)103-109
C. Hock, et al., Brain Research 755 (1997) 293-303
C. Hock, Neurobiology of Aging 19 (1998) 149-155
Koenig et al., J. Biol. Chem. 252 (1975) 2992-2997
McKhann, G., et al., Neurology 34 (1984) 939-944
Kretzschmar, et al., Nature (1996) 380: 345-347
Monnier and Cerami, Maillard Reaction in Food and Nutrition, ed. Waller, G. A., American Chemical Society (1983)431-448
Monnier and Cerami, Clinics in Endocrinology and Metabolism 11 (1982) 431-452
Tijssen, "Practice and theory of enzyme immunoassays", Elsevier, Amsterdam (1990)
Weidemann A., et al., Cell 57 (1989) 115-126
US 5,610,076
US 5,698,197

## Claims

1. Method of measurement of at least one advanced glycation endproduct (AGE) in a cerebrospinal fluid (CSF) sample, **characterized in that**, said CSF sample is pre-treated, wherein said pre-treatment results in protein denaturation.

2. Method according to claim 1, further **characterized in that**, said pre-treatment is performed using reagents selected from the groups of detergents, chaotropic reagents, and/or proteolytic enzymes.

3. Method according to any of claims 1 to 2, further **characterized in that**, said pre-treatment is performed using at least one proteolytic enzyme.

4. Method according to any of claims 1-3, further **characterized in that**, the proteolytic enzyme is proteinase K.

5. Method to assess a neurodegenerative disease, comprising comparing at least one AGE-CSF-level measured according to any of claims 1 - 4 to an AGE-CSF-level known or obtained from one or more CSF control samples.

6. Use of a method according to any of claims 1 - 5 to monitor Alzheimer's Disease.

7. Use of a method according to any of claims 1- 5 to diagnose Alzheimer's Disease.

8. Use of a method according to any of claims 1- 5 to screen for drugs influencing the level of an AGE in CSF.

9. Method to expose AGE-epitopes as present in a CSF sample, said method comprising use of a proteolytic enzyme, wherein that use of a proteolytic enzyme results in protein denaturation.

## Patentansprüche

1. Verfahren zum Messen zumindest eines fortgeschrittenen Glycosilierungsendprodukts (AGE) in einer Cerebrospinalflüssigkeits-(CSF)-Probe, **dadurch gekennzeichnet, dass** die CSF-Probe vorbehandelt wird, wobei die Vorbehandlung zur Proteindenaturierung führt.

2. Verfahren gemäß Anspruch 1, weiterhin **dadurch gekennzeichnet, dass** die Vorbehandlung unter Verwendung von Reagenzien durchgeführt wird, die ausgewählt sind aus der Gruppe von Detergenzien, chaotropen Reagenzien und/oder proteolytischen Enzymen.

3. Verfahren gemäß einem der Ansprüche 1 bis 2, weiterhin **dadurch gekennzeichnet, dass** die Vorbehandlung unter Verwendung zumindest eines proteolytischen Enzyms durchgeführt wird.

4. Verfahren gemäß einem der Ansprüche 1 bis 3, weiterhin **dadurch gekennzeichnet, dass** das proteolytische Enzym Proteinase K ist.

5. Verfahren zum Beurteilen einer neurodegenerativen Erkrankung, umfassend Vergleichen zumindest eines gemäß der Ansprüche 1 bis 4 gemessenen AGE-CSF-Spiegels mit einem bekannten oder aus einer oder mehrerer CSF-Kontrollproben erhaltenen AGE-CSF-Spiegel.

6. Verwendung eines Verfahrens gemäß einem der Ansprüche 1 bis 5, um die Alzheimer-Krankheit zu überwachen.

7. Verwendung eines Verfahrens gemäß einem der Ansprüche 1 bis 5, um die Alzheimer-Krankheit zu diagnostizieren.

8. Verwendung eines Verfahrens gemäß einem der Ansprüche 1 bis 5, um nach Medikamenten zu suchen, welche den Spiegel eines AGEs in CSF beeinflussen.

9. Verfahren zum Exponieren von AGE-Epitopen, wie sie in einer CSF-Probe vorliegen, wobei das Verfahren die Verwendung eines proteolytischen Enzyms umfasst, wobei diese Verwendung eines proteolytischen Enzyms zur Proteindenaturierung führt.

## Revendications

1. Procédé de mesure d'au moins un produit final de glycosylation approfondie (AGE) dans un échantillon de liquide céphalorachidien (CSF), **caractérisé en ce que** ledit échantillon de CSF est prétraité, dans lequel ledit prétraitement aboutit à une dénaturation des protéines.

2. Procédé selon la revendication 1, **caractérisé en outre en ce que** ledit prétraitement est réalisé en employant des réactifs choisis dans les groupes des détergents, des réactifs chaotropes, et/ou des enzymes protéolytiques.

3. Procédé selon l'une quelconque des revendications 1 à 2, **caractérisé en outre en ce que** ledit prétraitement est réalisé en employant au moins une enzyme protéolytique.

4. Procédé selon l'une quelconque des revendications 1 à 3, **caractérisé en outre en ce que** l'enzyme protéolytique est la protéinase K.

5. Procédé d'évaluation d'une maladie neurodégénérative, comprenant la comparaison d'au moins un taux AGE-CSF mesuré selon l'une quelconque des revendications 1 à 4 avec un taux AGE-CSF connu ou obtenu à partir d'un ou plusieurs échantillons témoins CSF.

6. Utilisation d'un procédé selon l'une quelconque des revendications 1 à 5 pour surveiller la maladie d'Alzheimer.

7. Utilisation d'un procédé selon l'une quelconque des revendications 1 à 5 pour diagnostiquer la maladie d'Alzheimer.

8. Utilisation d'un procédé selon l'une quelconque des revendications 1 à 5 pour dépister les médicaments influençant le taux d'un AGE dans du CSF.

9. Procédé pour révéler des épitopes de AGE, présents dans un échantillon CSF, ledit procédé comprenant l'utilisation d'une enzyme protéolytique, dans lequel cette utilisation d'une enzyme protéolytique aboutit à une dénaturation des protéines.
